**Europäisches Patentamt**

(19) **European Patent Office**

**Office européen des brevets**

(11) Veröffentlichungsnummer: **0 054 714**
**B1**

(12) # EUROPÄISCHE PATENTSCHRIFT

(45) Veröffentlichungstag der Patentschrift:
**28.01.87**

(51) Int. Cl.⁴: **F 16 L 37/12, A 61 M 16/00**

(21) Anmeldenummer: **81109185.9**

(22) Anmeldetag: **29.10.81**

(54) **Konnektions-System mit ineinandersteckbaren Verbindungselementen für Beatmungs- bzw. Anaestesie-Geräte.**

(30) Priorität: **20.12.80 DE 3048223**

(43) Veröffentlichungstag der Anmeldung:
**30.06.82 Patentblatt 82/26**

(45) Bekanntmachung des Hinweises auf die Patenterteilung:
**28.01.87 Patentblatt 87/5**

(84) Benannte Vertragsstaaten:
**AT BE CH DE FR GB IT LI LU NL SE**

(56) Entgegenhaltungen:
**EP - A - 0 018 440**
**BE - A - 718 873**
**DE - A - 2 628 359**
**DE - U - 6 750 287**
**DE - U - 7 920 347**
**FR - A - 478 920**
**FR - A - 2 442 393**
**US - A - 1 366 634**
**US - A - 3 670 726**
**US - A - 3 794 027**
**US - A - 4 275 907**

(73) Patentinhaber: **Rügheimer, Erich, Prof. Dr. med.,**
**Maximilianplatz 1, D-8520 Erlangen (DE)**

(72) Erfinder: **Rügheimer, Erich, Prof. Dr. med.,**
**Maximilianplatz 1, D-8520 Erlangen (DE)**

(74) Vertreter: **Hafner, Dieter Dr. Dipl.-Phys.,**
**Essenweinstrasse 4-6, D-8500 Nürnberg70 (DE)**

**Beschreibung**

Die Erfindung betrifft ein Konnektions-System mit ineinandersteckbaren Verbindungselementen für Beatmungs- bzw. Anästhesie-Geräte, wobei das eine Verbindungselement ein Y-Stück mit Einatmungsschlauchtülle, Ausatmungsschlauchtülle und Konnektoransatzstück und das andere Verbindungsstück ein mit dem Konnektoransatzstück verbindbarer Konnektor mit Tubusansatz für einen Endotrachealtubus ist.

Konnektions-Systeme dieser Art sind aus der US-A-3 794 027 bekannt und finden auch bei der künstlichen Beatmung von Patienten mit Hilfe einer Intubation Verwendung, beispielsweise zur Narkose mit Hilfe eines Anästhesie- oder Narkose-Gerätes vor und während einer Operation, aber auch zur langfristigen Dauerbeatmung von Patienten, beispielsweise auf Intensivstationen. Dabei ist ein Beatmungsgerät oder ein Narkosegerät über Einatem- bzw. Ausatemleitungen in Form von Gummi- oder Kunststoffrohren an ein Y-Stück angeschlossen, wobei die Rohre bzw. Schläuche auf eine Einatemschlauchtülle bzw. Ausatemschlauchtülle (Beine des Y-Stückes) aufgeschoben werden. Das Y-Stück weist ein Konnektoransatzstück auf, in das ein Konnektor mit einem Tubusansatz einsteckbar ist. Am Tubusansatz des Konnektors ist dann ein in die Luftröhre eines Patienten eingeführter Endotrachialtubus aufgeschoben.

Wesentlich ist es, dass die Verbindung zwischen Y-Stück und Konnektor bei Bedarf ausserordentlich rasch in einfacher Weise einhändig gelöst werden kann, während bei normalem Betrieb die Verbindung drehbeweglich bleiben soll, derart, dass der Konnektor innerhalb des Konnektoransatzstückes verdrehbar ist. Diese Steckverbindung ist bei bekannten Konnektions-Systemen durch eine konische Ausbildung des Endabschnittes des Konnektors und Innenöffnung des Konnektoransatzstückes im Sinne eines Feinschliffsitzes verwirklicht.

Bei derartigen Steckverbindungen besteht jedoch die Gefahr einer ungewollten und unerkannten Diskonnektion. Wird eine derartige Diskonnektion nicht oder nicht rechtzeitig erkannt, so kann das zu letalem Ausgang führen, wenn der Patient nicht mehr richtig bzw. gar nicht mehr beatmet wird.

Der Erfindung liegt daher die Aufgabe zugrunde, ein Konnektions-System der eingangs erläuterten Art, insbesondere zwischen einem intubierten Patienten und einem Beatmungs- bzw. Narkosegerät derart auszubilden, dass Undichtheiten vermieden werden, ungewollte Diskonnektionen mit Sicherheit ausgeschlossen sind, während gewollte und erforderliche Diskonnektionen in einfacher Weise, einhändig durchzuführen sind, wobei durch die Sicherung der Konnektion keine Verengung im Strömungsweg der Atemluft (oder $O_2$) bzw. des Narkosegases (z.B. Lachgas) auftreten dürfen. Das Konnektions-System soll auch in einfacher Weise sterilisierbar sein, wobei einzelne Verbindungselemente auch für einen einmaligen Gebrauch ausgebildet sein können, so dass aufwendige und mit Unsicherheiten behaftete Desinfektionsmassnahmen entfallen können.

Diese Aufgabe wird nach der Erfindung bei einem Konnektions-System der eingangs erläuterten Art dadurch gelöst, dass in der Aussenwandung des einsteckbaren Konnektors eine umlaufende ringförmige Nut vorgesehen ist, in die im gekuppelten Zustand des Konnektions-Systems mindestens ein am Konnektoransatzstück angeordnetes Sicherungselement unter Federwirkung elastisch eingreift, wobei die Verbindungselemente gegeneinander frei verdrehbar ausgebildet sind; dass das Sicherungselement als zweiarmiger, quer zur Längsachse des Konnektoransatzstückes verlaufender, um einen Zapfen verschwenkbarer und einseitig federbelasteter Hebel ausgebildet ist, der durch ein am Konnektoransatzstück einerseits und am äusseren Hebelarm andererseits angreifendes Federelement zum Eingriff mit seinem inneren Hebelarm in die Nut vorgespannt ist; dass ein handbetätigbares Entriegelungselement zur zeitweisen Überwindung der Federwirkung des Sicherungselementes vorgesehen ist; und dass am Einsteckende des Konnektors ein mit der Innenwandung des Konnektoransatzstückes zusammenwirkendes Dichtungselement vorgesehen ist.

Dadurch wird erreicht, dass das eine Verbindungselement mit dem zweiten Verbindungselement lediglich durch axiales Einschieben selbsttätig verriegelt wird, wobei gleichzeitig die erforderliche Dichtung zwischen den beiden Verbindungselementen hergestellt ist, um keine Störungen des Druckes und der Menge des durchströmenden Fluids auftreten zu lassen. Die Entriegelung bzw. Diskonnektion ist durch zeitweise Entfernung des Sicherungselementes, bei Überwindung der Schliessfederkraft, aus der Ringnut möglich.

Aus der DE-U-6 750 287 ist eine allgemein verwendbare Schlauchkupplung mit einer umlaufenden Entriegelungsnut und Dichtung im Steckerteil und einem federbelasteten Verriegelungshebel im Muffenteil schon bekannt. Diese Kupplung ist bevorzugt für Gartenschäuche entwickelt.

Gemäss einem bevorzugten Ausführungsbeispiel der Erfindung dient als Federelement eine Schraubenfeder, die in einer Innenbohrung am äusseren Hebelarm des Sicherungselementes und einer Innenbohrung im Konnektoransatzstück angeordnet ist.

Gemäss einem abgewandelten Ausführungsbeispiel des Sicherungselementes weist der äussere Hebelarm durch einen Schlitz abgetrennt einen federnden Steg als Federelement auf, der sich am Konnektoransatzstück abstützt.

Besonders vorteilhaft ist es, dass der äussere Hebelarm und/oder das Konnektoransatzstück eine Schrägfläche aufweist, die den Entriegelungsvorgang ermöglicht und eine Endbegrenzung für den Betätigungshub des äusseren Hebelarms des Sicherungselementes bildet.

Zur Erzielung der erforderlichen Dichtwirkung ist es vorteilhaft, wenn in weiterer Ausgestaltung der Erfindung die Wandstärke des vorderen in das Konnektoransatzstück einschiebbaren Endes des Konnektors konisch verjüngt ausgebildet ist und in eine Dichtlippe übergeht, die sich gegen einen schrägen Wandabschnitt der Innenwand des Konnektoransatzstückes elastisch federnd anlegt, wobei der erforderliche Anpressdruck durch die Verriegelung zwischen Nut und Sicherungselement der beiden Verbindungselemente bewirkt wird.

Zur Erleichterung der Anpressung einer Dichtlippe an den Wandabschnitt der Innenwand des Konnektoransatzstückes ist es vorteilhaft, wenn im Übergangsbereich zur Dichtlippe eine Aussenringnut vorgesehen ist.

In noch weiterer Ausgestaltung der Erfindung ist es vorteilhaft, dass die Dichtlippe einen geringeren Aussendurchmesser aufweist als der Konnektor und der Übergang vom Aussendurchmesser des Konnektors zum Durchmesser der Dichtlippe durch eine konische Schrägfläche gebildet ist, längs derer, beim Verbindungsvorgang der beiden Verbindungselemente das Sicherungselement gleitend in die Entriegelungslage gedrückt wird.

Bei wiederverwendbaren Konnektoren ist es zweckmässig, wenn das vordere in das Konnektoransatzstück einschiebbare Ende des Konnektors in einem Abschnitt von verringertem Rohrdurchmesser eine Ringnut mit eingelegter O-Ring-Dichtung aufweist.

Bei dem Konnektions-System gemäss der Erfindung können Y-Stücke Verwendung finden, bei denen der Konnektor gerade oder rechtwinklig angesetzt ist. Zur vorteilhaften Ausgestaltung, insbesondere zur Einweg-Verwendung ist es vorteilhaft, wenn mindestens der in das Y-Stück einführ- und verriegelbare Konnektor aus Kunststoff hergestellt ist.

In besonders vorteilhafter Weise ist, insbesondere für Einweg-Verwendung mit einem Konnektor ein aus Kunststoff gebildeter Endotrachealtubus thermisch verschweisst.

Gemäss einer weiteren Ausbildung des Konnektions-Systems nach der Erfindung für Beatmungs- bzw. Anästhesie-Geräte ist eine mit dem einen Y-Stück lösbar verbundene Kunstnase vorgesehen.

Gemäss einem bevorzugten Ausführungsbeispiel der Erfindung ist die Kunstnase zylinderrohrartig, mittels Schraub- oder Bajonettverschluss am Y-Stück aufsetzbar und an ihrem freien Ende als Konnektoransatz ausgebildet.

Dadurch ist es möglich, die Kunstnase mit dem Y-Stück in Verbindung mit einem beliebigen Konnektor oder aber auch mit einem Sicherheitskonnektor anzuordnen. Durch die Ausbildung der Verbindung als Schraub- oder Bajonettverschluss kann bei gleichbleibendem Y-Stück ein Konnektoransatzstück angeschraubt werden, zur Verbindung mit einem entsprechenden Konnektor.

Zweckmässigerweise trägt der Schraub- bzw. Bajonettverschlussansatz der Kunstnase eine in einer umlaufenden Ringnut angeordnete Linsen-Ringdichtung.

Vorteilhafterweise ist in der Kunstnase ein zylinderförmig ausgebildetes, metallbeschichtetes Kunststoffgitter zur Temperatur- und Feuchtigkeitsregelung der Atemluft angeordnet. Dabei ist das Kunststoffgitter mit einer Silbernitratauflage versehen. Anstelle des Kunststoffgitters kann jedoch auch ein Nickelsiebgitter oder dergl. Verwendung finden.

Vorteilhaft ist in der Kunstnase im Strömungswege der Atemluft vor und hinter dem metallbeschichteten Kunststoffgitter, je ein zylindrischer Abschnitt aus schwammartigem Schaumkunststoff angeordnet. Diese zylinderförmigen Abschnitte aus schwammartigem Schaumkunststoff dienen zur Regulierung der Feuchtigkeit der Atemluft.

Ein besonders vorteilhaftes Ausführungsbeispiel ist dadurch gekennzeichnet, dass das Kunststoffgitter und Schaumkunststoffabschnitte zu einer in die Kunstnase auswechselbar einsetzbaren Patrone ausgebildet sind. Dadurch ist der Ersatz der für die Regelung der Temperatur und Feuchtigkeit des Fluids notwendigen Teile in einfacher Weise ermöglicht, ohne dass das Gehäuse der Kunstnase bei jeder Erneuerung dieser Teile mit ausgewechselt werden muss.

Zur weiteren Sicherstellung der Dichtheit des Konnektions-Systems bei Beatmungs- bzw. Anästhesie-Geräten ist es vorteilhaft, dass die Einatmungsschlauchtülle und die Ausatmungsschlauchtülle des Y-Stückes schwach konisch ausgebildet sind und je mindestens einen sägezahnartigen Wulst aufweisen und dass die hintere, dem Konnektoransatzstück zugewandte Flanke des sägezahnartigen Wulstes steiler als die vordere Flanke jedoch zur Tüllenlängsachse unter einem Winkel kleiner als 90° geneigt ist.

Ferner ist es nach weiterer Ausgestaltung der Erfindung vorteilhaft, dass für die Atemschlauchtüllen teilweise umhüllende Klappen vorgesehen sind, die am Übergang zum Y-Stück mit Teilumfangsschlitzen versehen sind, und dass je ein axial verschiebbarer, umlaufender ovaler Klemmring für den zugeordneten Atemschlauch als Klemmittel dient.

Um den axial verschiebbaren umlaufenden Klemmring unverlierbar am Y-Stück zu befestigen, ist es vorteilhaft, dass die Klappen je eine vordere Randleiste und das Y-Stück je eine hintere Randleiste zur Begrenzung der axialen Verschiebung der Klemmringe aufweisen.

In noch weiterer Ausgestaltung des erfindungsgemässen Konnektions-Systems können am Y-Stück auswechselbare Bakterienfilter angeordnet sein. Die Anordnung kann dabei im Bereich der Atemschlauchtüllen, des Konnektoranschlussstutzens oder der Kunstnase-Patrone erfolgen.

Weitere Merkmale, Einzelheiten und Vorteile der Erfindung werden anhand der Zeichnung näher erläutert, die schematisch Ausführungsbeispiele der Erfindung ohne einschränkende Bedeutung darstellen.

Dabei zeigt:

Fig. 1 schematisch in perspektivischer Ansicht ein Ausführungsbeispiel eines erfindungsgemässen Konnektions-Systems,

Fig. 2a ein Y-Stück mit Konnektor als Verbindungselement in unverbundener Stellung,

Fig. 2b die Abwandlung eines Konnektors,

Fig. 2c eine weitere Abwandlung des Konnektors,

Fig. 3a einen Schnitt durch ein Y-Stück entlang der Linie A–A in Fig. 2a,

Fig. 3b eine Ansicht des Y-Stückes gemäss Fig. 3a mit eingeschobenem und verriegeltem Konnektor,

Fig. 4a ein Ausführungsbeispiel eines Y-Stükkes mit geradlinig angesetztem Konnektor,

Fig. 4b ein Detail eines Sicherungselementes,

Fig. 4c ein abgewandeltes Ausführungsbeispiel eines Sicherungselementes,

Fig. 5 ein abgewandeltes Y-Stück mit senkrecht angesetztem Konnektor, teilweise geschnitten,

Fig. 6 einen Teil-Schnitt des Ausführungsbeispiels nach Fig. 5, gesehen in Richtung des Pfeiles VI,

Fig. 7 ein Y-Stück mit einem Ansatzstück für eine Kunstnase,

Fig. 8 das Ausführungsbeispiel gemäss Fig. 7 mit einer Kunstnase mit einsetzbarer Patrone,

Fig. 9 ein abgewandeltes Ausführungsbeispiel eines Y-Stückes mit geschlitzten Klappen und gesicherten Atemschlauchtüllen,

Fig. 10 eine Ansicht des Ausführungsbeispieles nach Fig. 9 in Richtung des Pfeiles X,

Fig. 11 im oberen Bereich einen Schnitt durch ein Ausführungsbeispiel gemäss Fig. 9 und, im unteren Bereich, eine Draufsicht auf eine Klappe mit Klemmring.

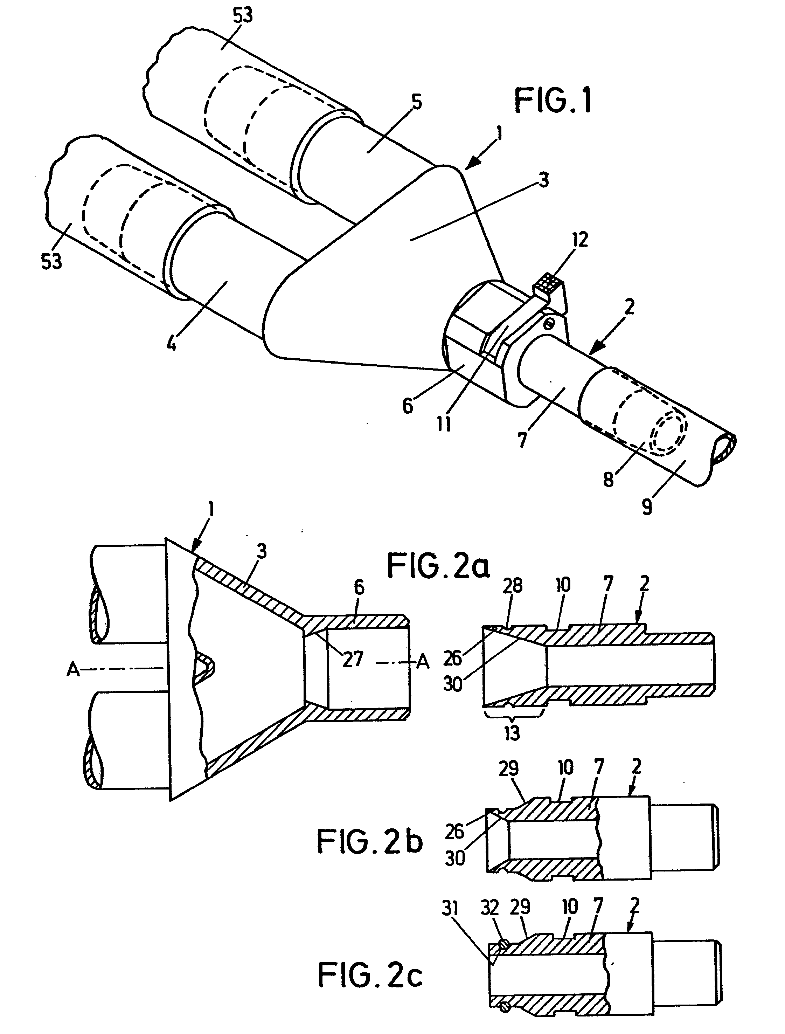

In Fig. 1 ist schematisch in perspektivischer Ansicht, teilweise geschnitten, ein Konnektions-System dargestellt, bestehend aus einem Verbindungselement 1 in Form eines Y-Stückes 3, einem zweiten Verbindungselement 2 in Form eines Konnektors 7 mit Tubusansatz 8, für beispielsweise einen Endotrachealtubus 9, sowie am Y-Stück 3 angeordnete Ein-Atemschlauchtülle 4 und Aus-Atemschlauchtülle 5, an die Atemschläuche 53 zur Verbindung mit beispielsweise einem Beatmungsgerät, einem Anästhesie- bzw. Narkosegerät angeschlossen sind. Das Y-Stück 3 weist ein Konnektoransatzstück 6 auf, in das der Konnektor 7 einschiebbar ist. Am Konnektoransatzstück 6 ist ein Sicherungselement 11 vorgesehen, mit einem Entriegelungselement 12. Der Aufbau des Sicherungselementes 11 und des Entriegelungselementes 12 und das Zusammenwirken mit dem zweiten Verbindungselement 2 wird später noch eingehend erläutert.

An die Verbindung der Verbindungselemente 1 und 2 werden, insbesondere bei der Anwendung für Atmungsgeräte oder dergl. besondere Anforderungen gestellt, insbesondere hinsichtlich relativer Verdrehbarkeit zueinander, sicherer Konnektion untereinander und wahlweiser Diskonnektion mit einer Hand sowie Verhinderung einer ungewollten Diskonnektion und ferner völlige Dichtheit zwischen den einzelnen Teilen des Konnektions-Systems untereinander, damit kein Entweichen von Atemluft oder dergl. und keine ungewollten Druckänderungen im System entstehen können. Bei Undichtheiten können Störungen des Fluid-Durchsatzes auftreten (z.B. Atemluft, Narkosegas oder dergl.) die zu Störungen bei der Beatmung eines Patienten und zu Alarmmeldungen an angeschlossenen Überwachungseinrichtungen führen können.

In Fig. 2a ist das aus dem Verbindungselement 1 in Form eines Y-Stückes 3 und dem Verbindungselement 2 in Form eines Konnektors 7 gebildete Konnektions-System in diskonnektierter Stellung im Schnitt dargestellt.

Bei dem Ausführungsbeispiel eines Konnektors 7 gemäss Fig. 2a ist an der Aussenwandung des Konnektors 7 eine umlaufende ringförmige Nut 10 angebracht. Der Konnektor 7 geht mit seinem vorderen, in das Y-Stück 3 bzw. in das Konnektoransatzstück 6 einführbarem Endbereich 13 konisch mit einer konischen Schrägfläche 30 in eine Dichtlippe 26 über. An der Aussenwandung der Dichtlippe 26 ist eine Aussenringnut 28 vorgesehen, um eine elastische Verformung der Dichtlippe 26 zu ermöglichen. Die Dichtlippe 26 wirkt, in eingeschobenem verriegeltem Zustand, mit einem analog geformten schrägen Wandabschnitt 27 im Konnektoransatzstück 6 zusammen.

Bei dem abgewandelten Ausführungsbeispiel des Konnektors 7 gemäss Fig. 2b ist zwischen der Ringnut 10 und der vorderen Schrägfläche 30 der Dichtlippe 26 eine Durchmesserstufe 29 vorgesehen, derart, dass das Sicherungselement 11 im Konnektoransatzstück 6 beim Einschieben des Konnektors 7 in das Konnektoransatzstück 6 aus seiner Verriegelungsstellung, an der Durchmesserstufe 29 entlanggleitend, in die Entriegelungsstellung gehoben wird. Beim weiteren Einschieben des Konnektors 7 in das Konnektoransatzstück 6 rastet dann das elastische Sicherungselement in die Ringnut 10 ein.

Die Ausführungsbeispiele des Konnektors 7 gemäss Fig. 2a und 2b sind vorzugsweise für Einmal-Konnektoren bestimmt, die nach der Beatmung eines Patienten nicht wieder verwendet werden und insbesondere aus Kunststoff hergestellt sind.

Das Ausführungsbeispiel gemäss Fig. 2c ist für wiederverwendbare Konnektoren 7 gedacht, wobei ebenfalls eine Durchmesserstufe 29 vorgesehen ist, jedoch anstelle der Dichtlippe 26 eine O-Ring-Dichtung 32 in eine Ringnut 31 eingelegt ist.

Durch die Anordnung der Dichtelemente, Dichtlippe 26 bzw. O-Ring-Dichtung 32 am Konnektor 7 werden Dichtungsmittel in der Innenöffnung des Konnektoransatzstückes 6 überflüssig. Dadurch entfällt einerseits die Schwierigkeit der Anbringung der Dichtungsmittel, andererseits die Schwierigkeit, die Dichtungsmittel nach Gebrauch zu sterilisieren. Ausserdem wird die Gefahr beseitigt, dass zwischen Verbindungselement 1 und Verbindungselement 2, d.h., Y-Stück 3 und Konnektor 7 keine ausreichende Dichtung erzielt wird, weil das Dichtungselement vergessen wurde oder beschädigt ist. Die einwandfreie Ausbildung der Dichtlippe 26 und das Vorhandensein der O-Ring- Dichtung 32 lassen sich vor Gebrauch in einfacher Weise überprüfen.

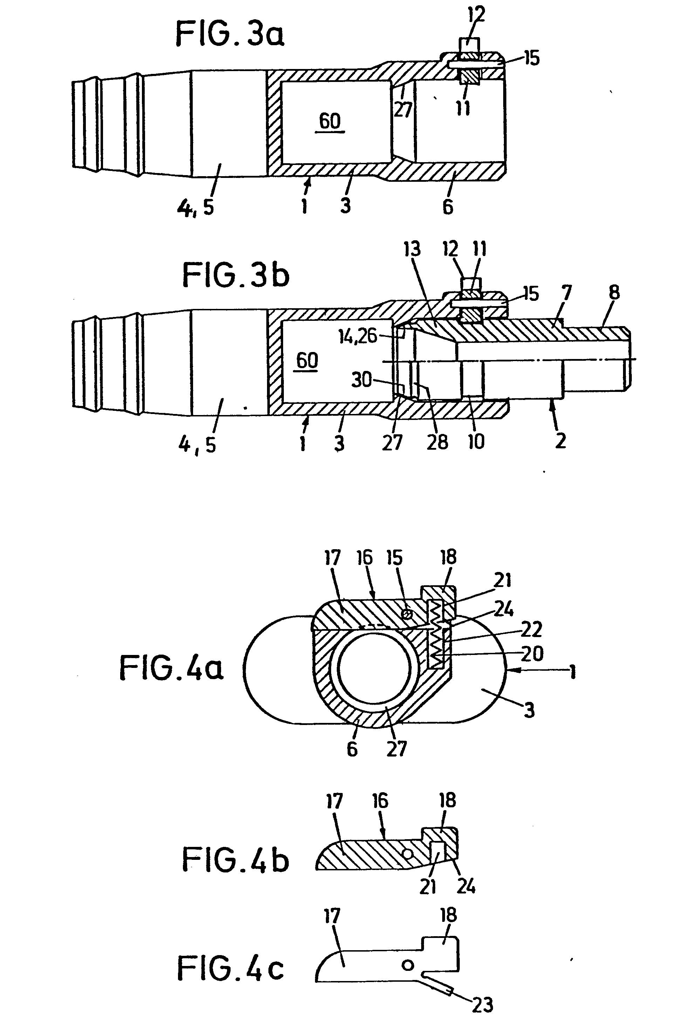

In Fig. 3a ist ein Schnitt durch ein Y-Stück gemäss Fig. 1 bzw. 2a längs der Ebene der Pfeile A–A in Fig. 2a dargestellt.

Das Konnektoransatzstück 6 geht, wie ersichtlich, in zwei Fenster 60 über, durch die die Verbindung zu den Atemschlauchtüllen 4 und 5 des Y-Stückes 3 hergestellt werden. Das Sicherungselement 11 ist um einen Zapfen 15 verschwenkbar gelagert und wird durch eine elastische Federwirkung in die in Fig. 3a gezeigte Verriegelungsstellung gedrückt. Zum Entriegeln wird das Sicherungselement 11 mit Hilfe des Entriegelungselementes 12 gegen die Wirkung der Feder angehoben und aus der Durchgangsöffnung der Innenöffnung im Konnektoransatzstück 6 gehoben, so dass ein eingeführter Konnektor 7 in einfacher Weise diskonnektiert werden kann.

Die Fig. 3b zeigt, ähnlich zur Fig. 3a, ein Y-Stück 3 mit eingeschobenem Konnektor 7. Die obere Hälfte der Fig. 3b zeigt einen Schnitt durch den Konnektor 7 und das Zusammenwirken des Sicherungselementes 11 mit der Ringnut 10. Die untere Hälfte zeigt das Verbindungselement 1, d.h. das Y-Stück 3, geschnitten und den Konnektor 7, d.h. das Verbindungselement 2, in Aussenansicht.

Aus Fig. 3b ist auch die Wirkung des als Dichtlippe 26 ausgebildeten Dichtungsmittels 14 am vorderen Ende des Konnektors 7 gezeigt. Die Dichtlippe 26 legt sich mit ihrer konischen Schrägfläche 30 gegen einen entsprechenden analog geformten konischen Wandabschnitt 27 der Innenöffnung des Konnektoransatzstückes 6 an. Durch diese Ausbildung wird erreicht, dass die Dichtlippe 26 durch den Innendruck im Leitungssystem fest dichtend an die Innenwand des Y-Stückes 3 bzw. Konnektoransatzstückes 6 gepresst wird.

In Fig. 4a ist das Y-Stück 3 im Schnitt durch das Konnektoransatzstück 6 auf Höhe des Sicherungselementes dargestellt. Dabei ist ersichtlich, dass das Sicherungselement 11 als zweiarmiger Hebel 16 mit einem inneren Hebelarm 17 und einem äusseren Hebelarm 18 ausgebildet ist. Der innere Hebelarm 17 bildet das Sicherungselement 11, welches in die Ringnut 10 des Konnektors 7 eingreift und die Verbindung verriegelt. Der Doppelarmhebel 16 ist um den Zapfen 15 verschwenkbar. Ein Federelement, beispielsweise eine Schraubenfeder 20 oder dergl., ist in einer Innenbohrung 21 des Hebels 16 und einer zugeordneten Innenbohrung 22 im Konnektoransatzstück 6 gelagert und drückt in der Darstellung gemäss Fig. 4a den Hebel 16 im Gegenzeigersinn mit dem inneren Hebelarm 17 nach unten. Der äussere Hebelarm 18 weist eine Schrägfläche 24 auf, die einerseits ein Kippen des Hebels 16 um den Zapfen 15 um einen bestimmten Winkelbereich ermöglicht und andererseits die Hubbewegung des Sicherungselements 11, d.h. des Hebelarmes 17, begrenzt.

In Fig. 4b ist das Sicherungselement 11 in Form des Hebels 16 getrennt dargestellt.

Die Fig. 4c zeigt eine Abwandlung eines Sicherungselementes 11 in Form eines zweiarmigen Hebels 16 mit innerem Hebelarm 17 und äusserem Hebelarm 18 aus Kunststoff, wobei am äusseren Hebelarm 18 als Federelement ein federnder Steg 23 angeformt ist. Der federnde Steg 23 übernimmt aufgrund seiner Elastizität die Wirkung des Federelementes 19 bzw. der Schraubfeder 20. Eine Schrägfläche, entsprechend der Schrägfläche 24, bei Ausführungsbeispiel gemäss Fig. 4a oder Fig. 4b muss bei diesem Ausführungsbeispiel an dem Konnektoransatzstück 6 vorgesehen sein.

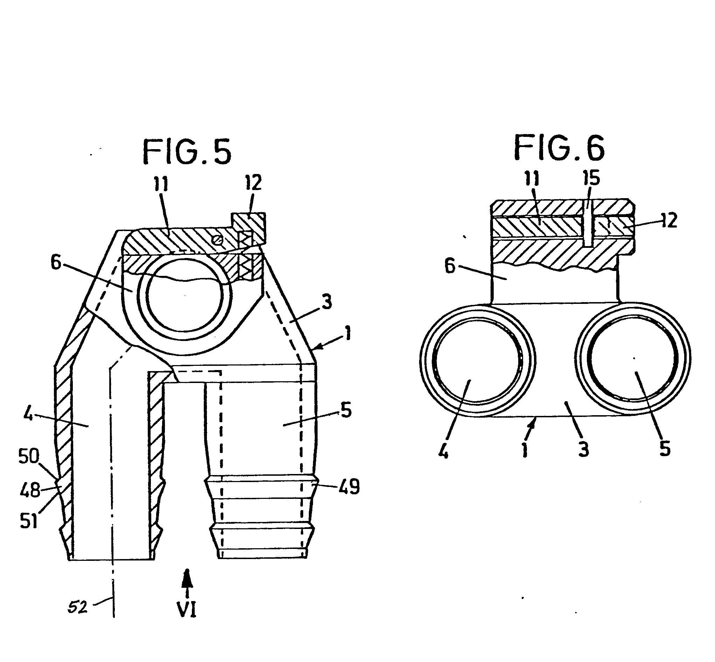

Bei den Ausführungsbeispielen der Fig. 1 bis 4 ist ein Y-Stück 3 dargestellt, an das das Konnektoransatzstück 6 gerade angesetzt ist. In den Fig. 5 und 6 ist ein abgewandeltes Ausführungsbeispiel dargestellt, bei dem das Konnektoransatzstück 6 und damit auch der Konnektor 7 an das Y-Stück 3 rechtwinklig angesetzt sind.

Das Sicherungselement 11 und Entriegelungselement 12 sind gleich aufgebaut, wie in den Fig. 4a bis 4c dargestellt.

Als weitere Sicherung des Konnektions-Systems ist in Fig. 5 am Verbindungselement 1, d.h. am Y-Stück 3 und insbesondere an dessen Atemschlauchtüllen 4 und 5 (Hosenrohren) noch mindestens je ein sägezahnartiger Wulst 48 bzw. 49 angeformt, der zur besseren Haftung der (nicht dargestellten) Atemschläuche dient. Durch diese Ausbildung wird der Tatsache Rechnung getragen, dass die Atemschläuche, beispielsweise einlageverstärkte Gummischläuche oder Kunststoffschläuche, nach mehrmaligem Gebrauch und Desinfektion altern und in ihrer Elastizität nachlassen. Durch die sägezahnartigen Wülste 48 und 49 ist sichergestellt, dass bei einer neuen Verbindung der Atemschläuche mit den Atemschlauchtüllen 4, 5 stets ein fester Sitz der Schläuche gewährleistet wird. Dabei ist die Ausbildung derart getroffen, dass die hintere Flanke 50 der sägezahnartigen Wülste 48 bzw. 49 steiler ist, als die vordere Flanke 51, jedoch unter einem Winkel kleiner als 90° zur Hosenrohr-(Tüllen)-Längsachse 52 geneigt sind.

Die Fig. 6 zeigt ein Y-Stück 3 mit rechtwinklig angesetztem Konnektoransatzstück 6 teilweise geschnitten, in Richtung des Pfeiles VI in Fig. 5 gesehen.

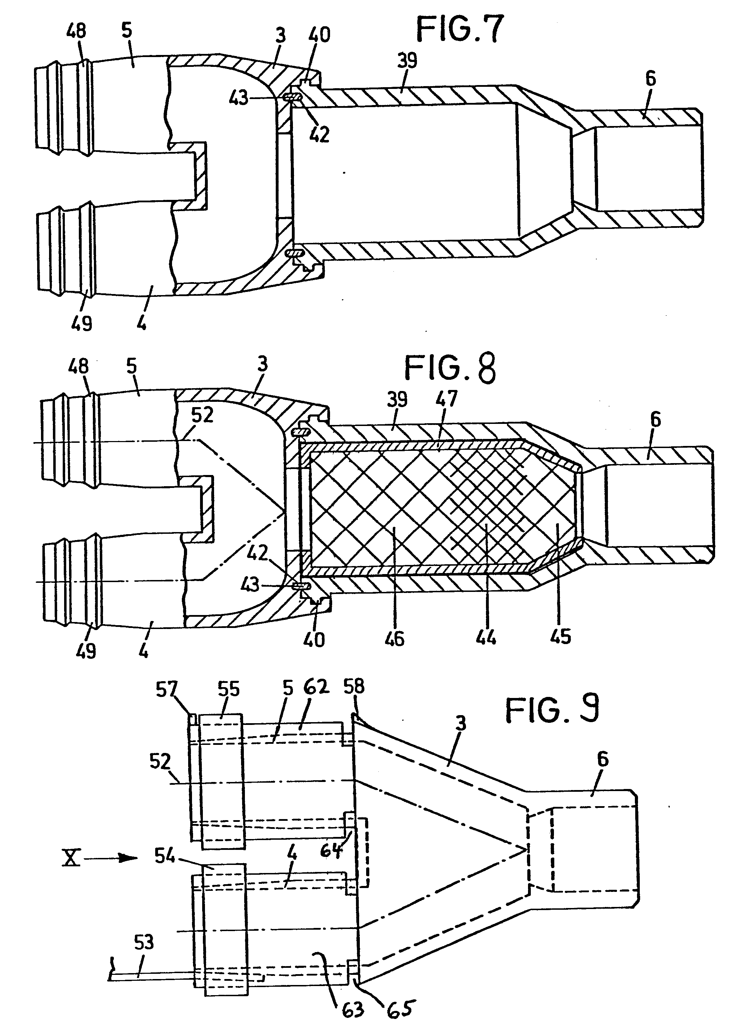

In Fig. 7 und 8 ist ein weiter abgewandeltes Ausführungsbeispiel dargestellt, mit einem Y-Stück 3, das mit Hilfe eines Schraub- bzw. Bajonettverschlusses 40 mit einer Kunstnase 39 verbindbar ist. In einer Ringnut 42 ist eine Linsen-Ringdichtung 43 angeordnet. Die Kunstnase 39 weist an ihrem, vom Y-Stück 3 abgewandten Ende das erforderliche Konnektoransatzstück 6 auf. Das Konnektoransatzstück 6 kann entsprechend den obenstehend beschriebenen Ausführungsbeispielen nach den Fig. 1 bis 6 ausgebildet sein.

Kunstnasen dienen bei Beatmungsgeräten bekanntlich dazu, die Feuchtigkeit und Temperatur der Atemluft zu beeinflussen, um den fehlenden Einfluss des Nasen-Rachenraumes des zu beatmenden Patienten auf den Zustand der Atemluft auszuregulieren.

In Fig. 8 ist ein Ausführungsbeispiel einer Kunstnase dargestellt, bei dem in der Kunstnase 39 eine Patrone 47 eingeführt ist, in der ein metallbeschichtetes, beispielsweise zylinderförmig ausgebildetes, Kunststoffgitter 44 vorgesehen ist. Als Beschichtung eignet sich insbesondere Silbernitrat. Anstelle des Kunststoffsiebes kann aber auch ein Nickelsiebgitter oder ein ähnlich wirkendes Element eingesetzt sein. Im Strömungsweg der Atemluft ist vor und hinter dem Kunststoffgitter 44 je ein zylinderförmiger Schaumkunststoffabschnitt 45 bzw. 46 angeordnet, wobei der Schaumkunststoff zur Regelung des Feuchtigkeitsgehaltes durch Absorption der Feuchtigkeit der Atemluft oder Beatmungsluft dient.

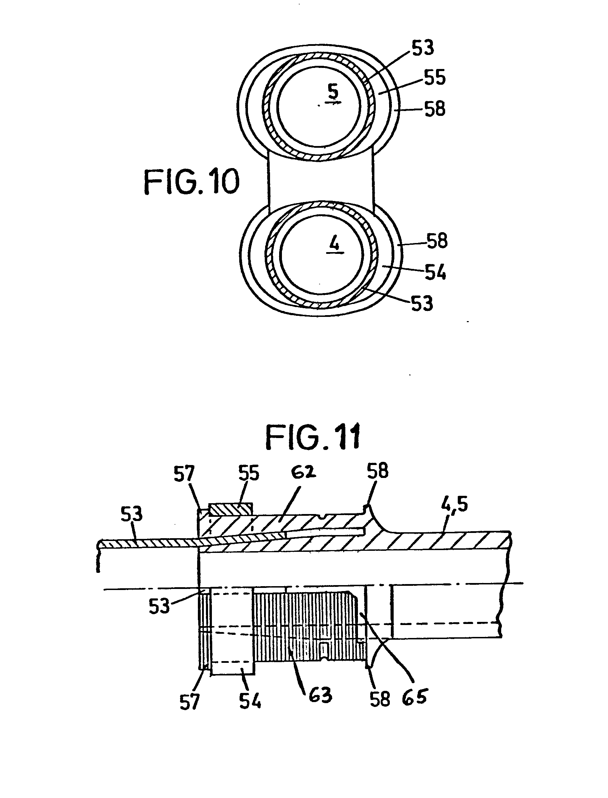

Die Kunstnase 39 mit Patrone 47 kann in Verbindung mit konventionellen Konnektoren Verwendung finden, andererseits können bei Y-Stücken 3 gemäss den Fig. 8 und 9 Konnektoransatzstücke 6 direkt mittels des Schraub- bzw. Bajonettverschlusses 40 mit dem Y-Stück 3 verbunden werden, ohne Zwischenanordnung einer Kunstnase 39.

In Fig. 9 ist ein abgewandeltes Ausführungsbeispiel einer Sicherung für Atemschläuche 53 an den Atemschlauchtüllen 4, 5 dargestellt. Dabei sind, was insbesondere bei Kunststoffausführung des Y-Stückes 3 in einfacher Weise möglich ist, jeweils die Atemschlauchtüllen 4 bzw. 5 teilweise umhüllende Klappen 62, 63 vorgesehen, die je einen ovalen Klemmring 54 bzw. 55 tragen, der parallel zur Tüllen-Längsachse 52 verschiebbar ist. Die ovale Ausbildung ist insbesondere deshalb vorteilhaft, da bei der räumlichen engen Nachbarschaft zwischen den Atemschlauchtüllen 4 und 5 die Anordnung eines runden Klemmringes einen erhöhten Platzbedarf bedeuten würde.

Um ein ungewolltes Lösen der Klemmringe 54 und 55 zu verhindern, ist es vorteilhaft, wenn an den Klappen 62, 63 eine vordere Randleiste 57 und am Y-Stück 3 eine hintere Randleiste 58 vorgesehen sind. Die ovalen Klemmringe 54 und 55 können, falls kein Atemschlauch eingeschoben ist, durch elastische Deformation der geschlitzten Atemschlauchtüllen 4 bzw. 5 aufgeschoben oder abgenommen werden. Die Fig. 10 zeigt ein Y-Stück gemäss Fig. 9 in Ansicht des Pfeiles X in Fig. 9.

Schliesslich zeigt Fig. 11 das Sicherheitssystem gemäss dem Ausführungsbeispiel nach Fig. 9 und 10 in Verbindung mit einem eingeschobenen oder aufgeschobenen Atemschlauch 53, im oberen Bereich geschnitten und in der unteren Hälfte in Seitenansicht.

Die Verbindungselemente 1 und 2, beispielsweise Y-Stück 3 und Konnektor 7, können bei den beschriebenen Ausführungsbeispielen der Erfindung sowohl aus Metall als auch, insbesondere für Einmalverwendung, aus Kunststoff hergestellt sein. Auch ist es möglich, eine Metall-Kunststoffkombination zu verwenden, indem beispielsweise das Y-Stück 3 aus Metall und der Konnektor 7 aus Kunststoff gefertigt werden. Bei der Kunststoffausführung ist es in vorteilhafter Weise möglich, den Tubus (Endotrachealtubus 9) mit dem Tubusansatz 8 des Konnektors 7 zu verschweissen, beispielsweise bei thermoplastischem Kunststoff durch entsprechende Wärmeanwendung. Die Verschweissung des Tubus 9 mit dem Konnektor 7 beseitigt eine weitere lösbare Verbindungsstelle, die zu ungewollten Diskonnektionen führen konnte. Die einstückige (verschweisste) Ausführung von Konnektor 7 und Endotrachealtubus 9 ermöglicht zusätzlich eine leichtere Handhabung und verringerte Lagerhaltung.

**Patentansprüche**

1. Konnektions-System mit ineinandersteckbaren Verbindungselementen (1, 2) für Beatmungs- bzw. Anästhesie-Geräte, wobei das eine Verbindungselement (1) ein Y-Stück (3) mit Einatmungsschlauchtülle (4), Ausatmungsschlauchtülle (5) und Konnektoransatzstück (6) und das andere Verbindungselement (2) ein mit dem Konnektoransatzstück (6) verbindbarer Konnektor (7) mit Tubusansatz (8) für einen Endotrachealtubus (9) ist, dadurch gekennzeichnet, dass in der Aussenwandung des einsteckbaren Konnektors (7) eine umlaufende ringförmige Nut (10) vorgesehen ist, in die im gekuppelten Zustand des Konnektions-Systems mindestens ein am Konnektoransatzstück (6) angeordnetes Sicherungselement (11) unter Federwirkung elastisch eingreift, wobei die Verbindungselemente (1, 2) gegeneinander frei verdrehbar ausgebildet sind; dass das Sicherungselement (11) als zweiarmiger, quer zur Längsachse des Konnektoransatzstückes (6) verlaufender, um einen Zapfen (15) verschwenkbarer und einseitig federbelasteter Hebel (16) ausgebildet ist, der durch ein am Konnektoransatzstück (6) einerseits und am äusseren Hebelarm (18) andererseits angreifendes Federelement (20, 23) zum Eingriff mit seinem inneren Hebelarm (17) in die Nut (10) vorgespannt ist; dass ein handbetätigbares Entriegelungselement (12) zur zeitweisen Überwindung der Federwirkung des Sicherungselementes (11) vorgesehen ist; und dass am Einsteckende (13) des Konnektors (7) ein mit der Innenwandung (27) des Konnektoransatzstückes (6) zusammenwirkendes Dichtungselement (26, 32) vorgesehen ist.

2. Konnektions-System nach Anspruch 1, dadurch gekennzeichnet, dass als Federelement eine Schraubenfeder (20) dient, die in einer Innenbohrung (21) am äusseren Hebelarm (18) des Sicherungselementes (11) und einer Innenbohrung (22) im Konnektoransatzstück (6) angeordnet ist.

3. Konnektions-System nach Anspruch 1, dadurch gekennzeichnet, dass der äussere Hebelarm (18) durch einen Schlitz abgetrennt einen federnden Steg (23) als Federelement aufweist, der sich am Konnektoransatzstück (6) abstützt.

4. Konnektions-System nach Anspruch 2 oder 3, dadurch gekennzeichnet, dass der äussere Hebelarm (18) und/oder das Konnektoransatzstück (6) eine Schrägfläche (24) aufweist, die den Entriegelungsvorgang ermöglicht und eine Endbegrenzung für den Betätigungshub des äusseren Hebelarms (18) des Sicherungselementes (11) bildet.

5. Konnektions-System nach einem der Ansprüche 1 bis 4, dadurch gekennzeichnet, dass die Wandstärke des vorderen in das Konnektoransatzstück (6) einschiebbaren Endes des Konnektors (7) konisch verjüngt ausgebildet ist und in eine Dichtlippe (26) übergeht, die sich gegen einen schrägen Wandabschnitt (27) der Innenwand des Konnektoransatzstückes (6) elastisch federnd anlegt, wobei der erforderliche Anpressdruck durch die Verriegelung zwischen Nut (10) und Sicherungselement (11) der beiden Verbindungselemente (1, 2) bewirkt wird.

6. Konnektions-System nach Anspruch 5, dadurch gekennzeichnet, dass im Übergangsbereich zur Dichtlippe (26) eine Aussenringnut (28) vorgesehen ist.

7. Konnektions-System nach Anspruch 5 oder 6, dadurch gekennzeichnet, dass die Dichtlippe (26) einen geringeren Aussendurchmesser aufweist als der Konnektor (7) und der Übergang vom Aussendurchmesser des Konnektors (7) zum Durchmesser der Dichtlippe (26) durch eine konische Schrägfläche (29) gebildet ist, längs derer, beim Verbindungsvorgang der beiden Verbindungselemente (1, 2) das Sicherungselement (11) gleitend in die Entriegelungslage gedrückt wird.

8. Konnektions-System nach einem der Ansprüche 1 bis 4, dadurch gekennzeichnet, dass das vordere in das Konnektoransatzstück (6) einschiebbare Ende des Konnektors (7) in einem Abschnitt von verringertem Rohrdurchmesser eine Ringnut (31) mit eingelegter O-Ring-Dichtung (32) aufweist.

9. Konnektions-System nach einem der Ansprüche 1 bis 8, dadurch gekennzeichnet, dass mindestens der in das Y-Stück (3) einführ- und verriegelbare Konnektor (7) aus Kunststoff hergestellt ist.

10. Konnektions-System nach Anspruch 9, dadurch gekennzeichnet, dass mit dem Konnektor (7) ein aus Kunststoff gebildeter Endotrachealtubus (9) thermisch verschweisst ist.

11. Konnektions-System nach einem der Ansprüche 1 bis 10, gekennzeichnet durch eine mit dem Y-Stück (3) lösbar verbundene Kunstnase (39).

12. Konnektions-System nach Anspruch 11, dadurch gekennzeichnet, dass die Kunstnase (39) zylinderrohrartig, mittels Schraub- oder Bajonettverschluss (40) am Y-Stück (3) aufsetzbar und an ihrem freien Ende als Konnektoransatz (6) ausgebildet ist.

13. Konnektions-System nach Anspruch 12, dadurch gekennzeichnet, dass der Schraub- bzw. Bajonettverschlussansatz (40) der Kunstnase (39) eine in einer umlaufenden Ringnut (42) angeordnete Linsen-Ringdichtung (43) trägt.

14. Konnektions-System nach einem der Ansprüche 11 bis 13, dadurch gekennzeichnet, dass in der Kunstnase (39) ein zylinderförmig ausgebildetes, metallbeschichtetes Kunststoffgitter (44) zur Temperatur- und Feuchtigkeitsregelung der Atemluft angeordnet ist.

15. Konnektions-System nach Anspruch 14, gekennzeichnet durch im Strömungsweg der Atemluft vor und hinter dem metallbeschichteten Kunststoffgitter (49) angeordnete zylindrische Abschnitte (45, 46) aus schwammartigem Schaumkunststoff.

16. Konnektions-System nach Anspruch 15, dadurch gekennzeichnet, dass das Kunststoffgitter (44) und die Schaumkunststoffabschnitte (45, 46) zu einer in die Kunstnasen (39) auswechselbar einsetzbaren Patrone (47) ausgebildet sind.

17. Konnektions-System nach einem der Ansprüche 1 bis 16, dadurch gekennzeichnet, dass die Einatmungsschlauchtülle (4) und die Ausatmungsschlauchtülle (5) des Y-Stückes (3) schwach konisch ausgebildet sind und je mindestens einen sägezahnartigen Wulst (48, 49) aufweisen und dass die hintere, dem Konnektoransatzstück (6) zugewandte Flanke (50) des sägezahnartigen Wulstes (48 bzw. 49) steiler als die vordere Flanke (51) jedoch zur Tüllenlängsachse (52) unter einem Winkel kleiner als 90° geneigt ist.

18. Konnektions-System nach einem der Ansprüche 1 bis 17, dadurch gekennzeichnet, dass für die Atemschlauchtüllen (4, 5) teilweise umhüllende Klappen (62, 63) vorgesehen sind, die am Übergang zum Y-Stück (3) mit Teilumfangsschlitzen (64, 65) versehen sind, und dass je ein axial verschiebbarer, umlaufender ovaler Klemmring (54, 55) für den zugeordneten Atemschlauch (53 bzw. 56) als Klemmittel dient.

19. Konnektions-System nach Anspruch 18, dadurch gekennzeichnet, dass die Klappen (62, 63) je eine vordere Randleiste (57) und das Y-Stück (3) je eine hintere Randleiste (58) zur Begrenzung der axialen Verschiebung der Klemmringe (54, 55) aufweisen.

20. Konnektions-System nach einem der Ansprüche 1 bis 19, dadurch gekennzeichnet, dass im Y-Stück (3) auswechselbar Bakterienfilter angeordnet sind.

**Claims**

1. Connection system comprising connecting elements (1, 2), which can be fitted together, for artificial respiration or anaesthesia apparatus, one connecting element (1) being a Y-tube (3) with an inhalation tube nozzle (4), an exhalation tube nozzle (5) and a connector extension piece (6) and the other connecting element (2) being a connector (7) which can be connected to the connector extension piece (6) and which has a tube extension for an endotracheal tube (9), characterised in that there is provided in the outer wall of the insertable connector (7) an encircling annular groove (10) in which at least one locking element (11) arranged on the connector extension piece (6) engages elastically under spring action when the connection system is coupled, the connecting elements (1, 2) being designed to be freely rotatable relative to one another; that the locking element (11) is in the form of a two-armed lever (16) which extends at right angles to the longitudinal axis of the connector extension piece (6), is pivotable about a pin (15) and is spring-loaded on one side and which is pretensioned by a spring element (20, 23), which bears against the connector extension piece (6) on the one hand and against the outer lever arm (18) on the other side, to engage in the groove (10) with its inner lever arm (17); that a manually operated unlocking element (12) is provided for the temporary overcoming of the spring force of the locking element (11); and that a sealing element (26, 32) which cooperates with the inner wall (27) of the connector extension piece (6) is provided at the insert end (13) of the connector (7).

2. Connection system according to Claim 1, characterised in that a helical spring (20) serves as the spring element which is arranged in an inner bore (21) on the outer lever arm (18) of the locking element (11) and an inner bore in the connector extension piece (6).

3. Connection system according to Claim 1, characterised in that the outer lever arm (18), which is divided by a slot, has a resilient web (23) as a spring element supported on the connector extension piece (6).

4. Connection system according to Claim 2 or 3, characterised in that the outer lever arm (18) and/or the connector extension piece (6) has an inclined face (24) which makes possible the unlocking action and forms an end limit for the operating lift of the outer lever arm (18) of the locking element (11).

5. Connection system according to any one of Claims 1 to 4, characterised in that the thickness of the front end of the connector (7) which can be inserted into the connector extension piece is designed to taper and leads into a sealing lip (26) which bears elastically and resiliently against an inclined wall section (27) of the inner wall of the connector extension piece (6), the required pressure being created by the locking action between the groove (10) and locking element (11) of the two connecting elements (1, 2).

6. Connection system according to Claim 5, characterised in that an outer annular groove (28) is provided in the transition area leading to the sealing lip (26).

7. Connection system according to Claim 5 or 6, characterised in that the sealing lip (26) has a smaller external diameter than the connector (7), and the transition area leading from the external diameter of the connector (7) to the diameter of the sealing lip (26) is formed by a conical inclined face (29) along which the locking element (11) is pressed with a sliding action into the unlocking position during the operation connecting the two connecting elements (1, 2).

8. Connection system according to any one of Claims 1 to 4, characterised in that the front end of the connector (7) which can be inserted into the connector extension piece (6) has an annular groove (31) with an inset O-ring seal (32) in a section of reduced tube diameter.

9. Connection system according to any one of Claims 1 to 8, characterised in that at least the connector (7) which can be inserted and locked into the Y-tube (3) is made of plastic material.

10. Connection system according to Claim 9, characterised in that an endotracheal tube (9) formed from plastic material is thermally welded to the connector (7).

11. Connection system according to any one of Claims 1 to 10, characterised by a synthetic projection (39) detachably connected to the Y-tube (3).

12. Connection system according to Claim 11, characterised in that the synthetic projection (39) is in the form of a cylindrical tube, can be attached to the Y-tube (3) by means of a screw-type or bayonet fitting (40) and is designed as a connector extension (6) at its free end.

13. Connection system according to Claim 12, characterised in that the screw-type or bayonet fitting extension (40) of the synthetic projection (39) carries a lenticular sealing ring (43) arranged in an encircling annular groove (42).

14. Connection system according to any one of Claims 11 to 13, characterised in that a cylindrically shaped metal-coated plastic mesh (44) for regulating the temperature and humidity of inhaled air is provided in the synthetic projection (39).

15. Connection system according to Claim 14, characterised by cylindrical sections (45, 46) made of sponge-like plastic foam and arranged in front of and behind the metal-coated plastic mesh (44) in the flow path of the inhaled air.

16. Connection system according to Claim 15, characterised in that the plastic mesh (44) and the plastic foam sections (45, 46) are designed to form a cartridge (47) which can be inserted interchangeably into the synthetic projections (39).

17. Connection system according to any one of Claims 1 to 16, characterised in that the inhalation tube nozzle (4) and the exhalation tube nozzle (5) of the Y-tube (3) are designed with a slight taper and each have at least one sawtooth-like bead (48, 49) and that the rear side (50) – facing the connector extension piece (6) – of the sawtooth-like bead (48 or 49) is steeper than the front side (51), but inclined at an angle of less than 90° to the longitudinal axis (52) of the nozzle.

18. Connection system according to any one of Claims 1 to 17, characterised in that there are provided for the breathing tube nozzles (4, 5) partially envelopping flaps (62, 63) which, in the transition area leading to the Y-tube (3), are provided with partial circumferential slots (64, 65), and that each axially slidable, encircling oval clamping ring (54, 55) serves as a means of clamping the associated breathing tube (53 or 56).

19. Connection system according to Claim 18, characterised in that the flaps (62, 63) each have a front edge strip (57) and the Y-tube (3) has a rear edge strip (58) to limit the axial movement of the clamping rings (54, 55).

20. Connection system according to any one of Claims 1 to 19, characterised in that interchangeable bacteria filters are provided in the Y-tube (3).

**Revendications**

1. Système de connexion comportant des éléments de liaison (1, 2), pour appareils respiratoires ou d'anesthésie, qui sont emboîtables l'un dans l'autre et dont l'un (1) est une pièce en Y (3) présentant un embout (4) pour tuyau souple d'inspiration, un embout (5) pour tuyau souple d'expiration et une pièce de prolongement (6) pour connecteur alors que l'autre élément de liaison (2) est un connecteur (7) pouvant être assemblé à la pièce de prolongement (6) et présentant un embout (8) pour un tube endotrachéal (9), caractérisé en ce que la paroi externe du connecteur emboîtable (7) est munie d'une gorge annulaire continue (10) dans laquelle, à l'état accouplé du système de connexion, au moins un élément d'arrêt (11) disposé sur la pièce de prolongement (6) vient en prise élastiquement, sous l'effet d'une action élastique, les éléments de liaison (1, 2) étant agencés de manière à pouvoir tourner librement l'un par rapport à l'autre; en ce que l'élément d'arrêt (11) est agencé sous la forme d'un levier (16) à deux bras, s'étendant transversalement à l'axe longitudinal de la pièce de prolongement (6), pouvant basculer autour d'un doigt (15) et chargé élastiquement d'un côté, lequel levier est précontraint, en vue d'être mis en prise dans la gorge (10) par son bras intérieur (17), à l'aide d'un élément élastique (20, 23) prenant appui d'une part sur la pièce de prolongement (6) et d'autre part sur le bras de levier extérieur (18); en ce qu'il est prévu un élément de déverrouillage (12) manœuvrable à la main et servant à vaincre temporairement l'action élastique de l'élément d'arrêt (11); et en ce qu'un élément d'étanchéité (26, 32), coopérant avec la paroi interne (27) de la pièce de prolongement (6), est prévu à l'extrémité à emboîter (13) du connecteur (7).

2. Système de connexion selon la revendication 1, caractérisé en ce que l'élément élastique est constitué par un ressort hélicoïdal (20) qui est disposé dans un alésage intérieur (21) réalisé sur le bras de levier extérieur (18) de l'élément d'arrêt (11) et dans un alésage intérieur (22) réalisé dans la pièce de prolongement (6).

3. Système de connexion selon la revendication 1, caractérisé en ce que le bras de levier extérieur (18) comporte, en tant qu'élément élastique, une ailette jouant élastiquement (23), séparée par une fente et s'appuyant sur la pièce de prolongement (6).

4. Système de connexion selon la revendication 2 ou 3, caractérisé en ce que le bras de levier extérieur (18) et/ou la pièce de prolongement (6) présentent une surface inclinée (24) qui permet l'opération de déverrouillage et constitue une limite extrême pour la course de manœuvre du bras de levier extérieur (18) de l'élément d'arrêt (11).

5. Système de connexion selon l'une des revendications 1 à 4, caractérisé en ce que la paroi de l'extrémité avant du connecteur (7), à insérer dans la pièce de prolongement (6), a son épaisseur qui est agencée de manière à se rétrécir coniquement et qui évolue en formant une lèvre d'étanchéité (26), laquelle s'appuie élastiquement à la manière d'un ressort contre un tronçon incliné (27) de la paroi interne de la pièce de prolongement (6), la pression d'appui nécessaire étant assurée par le verrouillage entre la gorge (10) et l'élément d'arrêt (11) des deux éléments de liaison (1, 2).

6. Système de connexion selon la revendication 5, caractérisé en ce qu'une gorge annulaire externe (28) est prévue dans la zone d'évolution vers la lèvre d'étanchéité (26).

7. Système de connexion selon la revendication 5 ou 6, caractérisé en ce que la lèvre d'étanchéité (26) présente un diamètre extérieur plus faible que le connecteur (7) et en ce que l'évolution du diamètre extérieur du connecteur (7) vers le diamètre de la lèvre d'étanchéité (26) est constituée par une surface inclinée conique (29) le long de laquelle, lors de l'opération d'assemblage des deux éléments de liaison (1, 2), l'élément d'arrêt (11) se trouve repoussé en glissant vers la position de déverrouillage.

8. Système de connexion selon l'une des revendications 1 à 4, caractérisé en ce que l'extrémité avant du connecteur (7), à introduire dans la pièce de prolongement (6), comporte, dans un tronçon de diamètre de tube réduit, une gorge annulaire (31) dans laquelle est inséré un joint d'étanchéité torique (32).

9. Système de connexion selon l'une des revendications 1 à 8, caractérisé en ce qu'au moins le connecteur (7), à introduire et verrouiller dans la pièce Y (3), est fabriqué en matière plastique.

10. Système de connexion selon la revendication 9, caractérisé en ce qu'un tube endotrachéal (9), réalisé en matière plastique, est soudé thermiquement au connecteur (7).

11. Système de connexion selon l'une des revendications 1 à 10, caractérisé par un nez artificiel (39) relié de manière amovible à la pièce en Y (3).

12. Système de connexion selon la revendication 11, caractérisé en ce que le nez artificiel (39), en forme de tube cylindrique, est adaptable sur la pièce en Y (3) au moyen d'une fixation à baïonette ou vissée (40) et est agencé à son extrémité libre sous la forme d'un embout pour connecteur (6).

13. Système de connexion selon la revendication 12, caractérisé en ce que l'embout de fixation à baïonette ou vissée (40) du nez artificiel (39) porte un joint d'étanchéité annulaire (43), en forme de lentille, qui est disposé dans une gorge annulaire continue (42).

14. Système de connexion selon l'une des revendications 11 à 13, caractérisé en ce que le nez artificiel (39) est muni d'une grille de matière plastique (44), revêtue de métal, agencée en forme de cylindre et servant au réglage de la température et de l'humidité de l'air à respirer.

15. Système de connexion selon la revendication 14, caractérisé en ce que des tronçons cylindrique (45, 46), en matière plastique cellulaire spongieuse, sont disposés sur le trajet d'écoulement de l'air à respirer, en amont et en aval de la grille de matière plastique revêtue de métal (44).

16. Système de connexion selon la revendication 15, caractérisé en ce que la grille de matière plastique (44) et les tronçons de matière plastique cellulaire (45, 46) sont agencés sous la forme d'une cartouche (47) pouvant être insérée de manière interchangeable dans le nez artificiel (39).

17. Système de connexion selon l'une des revendications 1 à 16, caractérisé en ce que l'embout pour tuyau souple d'inspiration (4) et l'embout pour le tuyau souple d'expiration (5) de la pièce en Y (3) sont agencés de manière faiblement conique et comportent chacun au moins un bourrelet en dent de scie (48, 49), et en ce que le flanc arrière (50), tourné vers la pièce de prolongement pour connecteur (6), du bourrelet en dent de scie (48 ou 49) est plus raide que le flanc avant (51), mais est toutefois incliné par rapport à l'axe longitudinal d'embout (52) d'un angle plus petit que 90°.

18. Système de connexion selon l'une des revendications 1 à 17, caractérisé en ce que les embouts pour tuyau souple respiratoire (4, 5) comportent des volets qui les enveloppent partiellement (62, 63) et qui sont munis, au niveau de la transition avec la pièce en Y (3), de fentes (64, 65) ne s'étendant que sur une partie de la périphérie, et en ce qu'une bague ovale de serrage (54, 55) continue, déplaçable en translation axiale, sert de moyen de serrage pour chacun des tuyaux souples respiratoires associés (53 ou 56).

19. Système de connexion selon la revendication 18, caractérisé en ce que, pour limiter le déplacement en translation axiale de chacune des bagues de serrage (54, 55), les volets (62, 63) comportent une nervure de bord antérieure (57) et la pièce en Y (3) comporte une nervure de bord postérieure (58).

20. Système de connexion selon l'une des revendications 1 à 19, caractérisé en ce que des filtres à bactéries sont disposés de manière amovible dans la pièce en Y (3).

FIG.1

FIG.2a

FIG.2b

FIG.2c

FIG.3a

FIG.3b

FIG.4a

FIG.4b

FIG.4c

FIG.5

FIG.6

FIG.7

FIG.8

FIG.9

FIG.10

FIG.11